# EUROPEAN PATENT APPLICATION

(11) **EP 3 643 222 A1**
(43) Date of publication of application: **29.04.2020**
(21) Application number: 18202736.7
(22) Date of filing: 26.10.2018
(51) Int. Cl.: A61B 5/00, A61B 5/02, A61B 5/0215, A61B 5/026

(54) **DEVICE AND SYSTEM FOR ASSESSING RELIABILITY OF VESSEL PHYSIOLOGY RELATED MEASUREMENT**

(71) Applicant: Koninklijke Philips N.V., 5656 AG Eindhoven (NL)
(72) Inventor: SIO, Charles Frederik, 5656 AE Eindhoven (NL); KUENEN, Maarten Petrus Joseph, 5656 AE Eindhoven (NL); VAN DER HORST, Arjen, 5656 AE Eindhoven (NL)
(74) Representative: de Haan, Poul Erik

(57) **Abstract**

The present invention relates to vessel physiology related measurements. A device is provided in order to improve workflow, e.g. in view of measurements in an interventional procedure performed in a cathlab. The device comprises an input unit, a processing unit and an output unit. The input unit is configured to receive at least one vessel physiology related measurement from a detection device inserted into the vessel; and to receive location information of the detection device, which location information is related to a position of the detection device inside the vessel during the vessel physiology related measurement. The processing unit is configured to determine a reliability factor for the vessel physiology related measurement based on the location information. The output unit is configured to provide the reliability factor for the vessel physiology related measurements.

## Description

### FIELD OF THE INVENTION

The present invention relates to devices, systems and a method for vessel physiology related measurements.

### BACKGROUND OF THE INVENTION

Hypertension and its associated conditions, chronic heart failure and chronic renal failure, constitute a significant and growing global health concern. Current therapies for these conditions include non-pharmacological, pharmacological, surgical, and implanted device-based approaches. Renal denervation is a treatment option for resistant and uncontrolled hypertension. However, the efficacy of renal denervation may be very variable between patients. It has been shown that the arterial stiffness of the main renal artery and renal blood flow/resistance before the procedure may be indicative of the outcome of a renal denervation therapy procedure. Hence, efforts are undertaken to determine the stiffness of the main renal arteries for renal denervation patient stratification. One approach is to determine blood pressure at different locations. Another approach is to determine pulse wave velocity (PWV) inside the main renal artery. For example, WO 2017/198490 A1 relates to determining the velocity of the pressure/flow pulse, i.e. pulse wave velocity inside the main renal artery, for further improving the stratification of patients in view of the outcome of renal denervation.

However, it has been shown that the quality of the measurements, and thus the accuracy and reliability of the calculated renal arterial stiffness, may be cumbersome to assess by a physician.

### SUMMARY OF THE INVENTION

Since the measurement is an interventional procedure performed in a cathlab comprising catheterization of the renal artery, and the subsequent renal denervation treatment is also an interventional procedure performed in a cathlab comprising catheterization of the renal artery, a need exists to provide an improved and facilitated workflow.

The object of the present invention is solved by the subject-matter of the independent claims; further embodiments are incorporated in the dependent claims. It should be noted that the following described aspects of the invention apply also for the device for renal artery measurement, for the system for renal artery measurement and for the method for renal artery measurement.

According to the present invention, a device for renal artery measurement is provided. The device comprises an input unit, a processing unit and an output unit. The input unit is configured to receive at least one measured renal artery related parameter from a detection device inserted in the renal artery. The input unit is further configured to receive location information of the detection device, which location information is related to a position of the detection device inside the renal artery during the measuring. The processing unit is configured to determine a reliability factor for the measured parameter based on the location information. Further, the output unit is configured to provide the reliability factor for the measured at least one renal artery related parameter.

This results in a valuable feedback concerning the quality of the measured data which can then be considered for further steps.

According to the present invention, also a system for renal artery measurement is provided. The system comprises a device for renal artery measurement according to the example above. Further, a measurement arrangement comprising a detection device for insertion into a renal artery of a subject is provided, the detection device having at least one sensor configured to measure and to provide the at least one renal artery related parameter.

The term "subject" may also be referred to as individual. The "subject" may further also be referred to as patient, although it is noted that this term does not indicate whether any illness or disease is actually present with the subject.

According to an example, it is further provided a medical imaging system configured to acquire medical image data during the measuring of the at least one renal artery related parameter. Further, at least one marker for the at least one sensor is provided, which marker is detectable by the medical imaging system. The processing unit is configured to determine the location of the at least one sensor in relation to the renal artery based on the medical image data. The processing unit is also configured to compare the determined location to at least one predefined location threshold for the reliability factor.

In an option, the medical imaging system is an X-ray imaging system and the at least one marker is visible in X-ray images, e.g. radiopaque.

In another option, the medical imaging system is an ultrasound imaging system and the at least one marker is visible in ultrasound.

According to an example, the detection device comprises an ultrasound transducer provided as an active marker that detects, e.g. picks up, ultrasound waves from an external ultrasound probe.

According to an example, the detection device comprises two pressure sensors spaced apart with a predetermined distance. A marker is provided for each of the two pressure sensors, which markers are detectable by the medical imaging system. The processing unit is configured to determine a projected distance of the two pressure sensors in relation to a central axis of the renal artery based on the medical image data. Further, the processing unit is configured to compare the projected distance with the predetermined distance for the reliability factor.

According to an example, the detection device comprises a flow sensor, for example a flow sensor or a flow velocity sensor. A marker is provided for the flow sensor, which marker is detectable by the medical imaging system. The processing unit is configured to determine, based on the medical image data acquired during the measuring of the at least one renal artery related parameter, a relative distance of the flow sensor in relation to a central axis of the renal artery and/or in relation to a wall of the renal artery based on the medical image data. The processing unit is also configured to compare the relative distance with a predetermined distance value for the reliability factor.

According to an example, the detection device comprises a flow sensor and at least one sensor that measures distance values. The processing unit is configured to determine a distance of the flow sensor to the renal artery wall during the flow measurement; and to compare the distance with a predetermined distance value.

According to an example, a medical imaging system is provided that acquires medical image data during the measuring of the at least one renal artery related parameter. The medical image data comprises structural anatomical information of the renal artery comprising at least one of the group of branching off location from the aorta, side branches and connecting location with the glomerulus. The processing unit is configured to determine the location of the at least one sensor in relation to the renal artery based on the medical image data. The processing unit is also configured to compare the determined location to at least one predefined location for the reliability factor.

According to an example, the measurement arrangement is configured to measure over a period of time that incorporates a predetermined number of heartbeats. The measurement arrangement is further configured to provide measurements for sub-periods of the period of time. The processing unit is configured to determine a variation between measurements of different sub-periods, and to compare the variation to at least one predefined variation threshold.

According to an example, the measurement arrangement is configured to perform two measurements. The processing unit is configured to determine a variation of the values for the two measurements, and to compare the variation to at least one predefined variation threshold.

According to an example, three sensors are provided comprising pressure sensors and/or flow sensors. The processing unit is configured to calculate a renal artery related parameter based on different combinations of the three sensors. The processing unit is also configured to determine a variation of the calculated pulse wave velocities and to compare the variation to at least one predefined variation threshold.

According to the present invention, also a method for renal artery measurement is provided. The method comprises the following steps:
a) measuring at least one renal artery related parameter with a detection device inserted in the renal artery;
b) providing location information of the detection device, which location information is related to a position of the detection device inside the renal artery during the measuring;
c) determining a reliability factor for the measuring based on the location information; and
d) providing the reliability factor for the measured at least one renal artery related parameter.

According to an aspect, image and signal analysis is provided to determine the reliability of the arterial stiffness measurement. A renal artery measurement that is provided by one or more sensors is combined with a location information, which then leads to the verification or reliability assessment.

The device for renal artery measurement, respectively system and method, as described above and below, provide guidance for the physician, and analysis of the quality and reliability of the outcome of the renal artery stiffness measurement, to ensure that clinical decisions are based on correct measurement outcomes. This provides an improved and facilitated workflow which is in particular of advantage during the time that's available in a typical procedure.

In an option, the intravascular device with sensors to determine arterial stiffness, PWV, blood flow, and/or resistance, is combined with a software to identify and position the intravascular device in separate medical imaging, e.g. X-ray or ultrasound, to determine the position of the sensors, to segment the renal artery, and to calculate the position of the sensors with respect to the renal artery. Another option provides radiopaque markers on the intravascular device for a more exact position detection in X-ray images. A further option provides ultrasound markers on the intravascular device, passive or active, e.g. in situ, i.e. on the device inside the vascular structure at or close to the location of the sensor, for a more exact position detection in ultrasound images. A still further option provides sensors, e.g. IVUS or OCT, on the intravascular device to determine positioning of the intravascular device with respect to the renal artery. A software-based option is to analyze the reproducibility of the measurement outcome over a number of heartbeats. A further software-based option is to analyze the dynamic signals, e.g. spectrum, from the sensors over a number of heartbeats. In another option, a pull-back device is provided to allow comparison over time and location. The pull-back device is thus starting distal and then advancing the wire proximally while measuring. For example, PWV is assessed at different positions in the renal artery and consistency is checked between the different PWV values. A further option is a push-forward device provided to allow comparison over time and location. The push-forward device is thus a reverse way by starting proximal and advancing the wire distally while measuring. Another option provides to assess the quality of the measurements based on the elements above and provide guidance whether the generated arterial stiffness / PWV outcome should be used for patient stratification.

The present invention is exemplarily described with reference to renal artery related measurements, however, the invention can be used in other clinical applications for vessel physiology related measurements, e.g. coronary vessels, vessels of other organs such as kidney and liver, peripheral vessels of the limbs.

The term vessel physiology related measurements comprise at least one of the following measurements: volumetric blood flow, blood flow velocity, vessel resistance, blood pressure, pulse wave velocity, vessel wall stiffness, vessel wall compliance and the change of any of the aforementioned due to a stimulus. The vessel physiology related measurements are referred to or are equivalent in the detailed description with parameters determined from the vessel physiology related measurements.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a device for renal artery measurement.
Fig. 2 shows an example of a system for renal artery measurement, comprising the device for renal artery measurement of Fig. 1 and a detection device for insertion into a renal artery of a subject.
Fig. 3 shows an extract of an example of a system for renal artery measurement comprising an X-ray imaging system.
Fig. 4 shows an extract of a further example of a system for renal artery measurement comprising an ultrasound imaging system.
Fig. 5 illustrates an example of the detection device for insertion into a renal artery of a subject in the context of a renal artery branching off from an aorta towards a kidney.
Fig. 6 shows a further example of a system for renal artery measurement.
Fig. 7 shows an extract of a further example of a system for renal artery measurement.
Fig. 8 shows an extract of a still further example of a system for renal artery measurement with a medical imaging system.
Fig. 9 shows a further example of a system for renal artery measurement.
Fig. 10 shows a comparison of flow signals according to another example of a system for renal artery measurement.
Fig. 11 shows a further comparison of flow signals according to a further example of a system for renal artery measurement.
Fig. 12 shows steps of an example of a method for renal artery measurement.

### DETAILED DESCRIPTION OF EMBODIMENTS

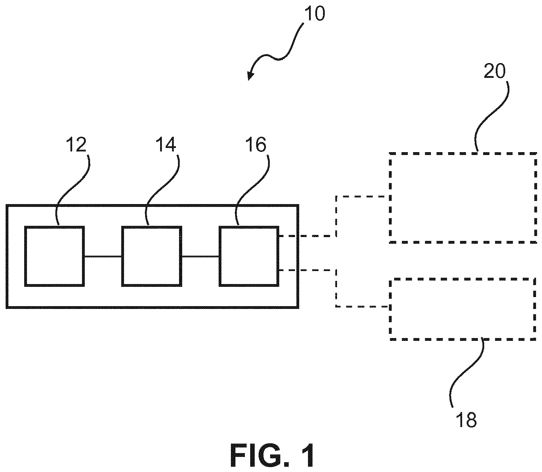

Fig. 1 shows an example of a device 10 for renal artery measurement. The device 10 comprises an input unit 12, a processing unit 14 and an output unit 16. The input unit 12 is configured to receive at least one measured renal artery related parameter from a detection device inserted in the renal artery. The input unit 12 is also configured to receive location information of the detection device, which location information is related to a position of the detection device inside the renal artery during measurement. The processing unit 14 is configured to determine a reliability factor for the measured parameter based on the location information. The output unit 16 is configured to provide the reliability factor for the measured at least one renal artery related parameter.

As an example, the term renal related parameter measurement or renal vessel physiology related measurement comprises at least one of the following measurements: volumetric blood flow, blood flow velocity, renal resistance, blood pressure, pulse wave velocity, vessel wall stiffness or change of any of the aforementioned due to a stimulus. A stimulus can be provided e.g. by vasoactive drugs, handgrip and electrical.

In an example, the at least one renal artery related parameter comprises at least a flow measurement of blood flow in the renal artery.

In an example, shown as an option in Fig. 1 with hashed lines, a console 18 is provided as a user interface for operating the device. As a further option also shown with hashed lines, the output unit 16 further comprises display 20. As an example, the reliability factor is shown on the display 20. In an option, the device comprises only a display 20, but not a console. In a further option, the device comprises only a console 18, but not a display.

Renal artery related parameters may relate to the stiffness of the vessel or to other parameters like size and (current) blood pressure and blood flow. For measuring renal artery related parameter, as an example, two pressure sensors are provided. In another example, pressure and flow measurements are provided. In a further example, also the diameter of a vessel is measured. For flow measurement, the center portion of the vessel is used for positioning the sensor. In an example, for pressure and also flow, two sensors are placed in a distance along the vessel, i.e. along in terms of flowing direction. Further, positioning also relates to anatomical aspects, such as the location of the sensors in the correct depth.

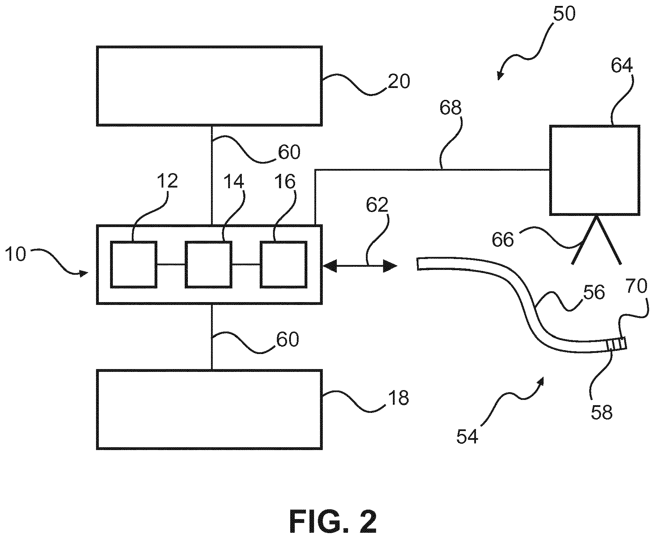

Fig. 2 shows an example of a system 50 for renal artery measurement. The system 50 comprises an example of the device 10 for renal artery measurement according to one of the examples above. Further, a measurement arrangement 54 is provided comprising a detection device 56 for insertion into a renal artery of a subject, the detection device having at least one sensor 58 configured to measure and to provide the at least one renal artery related parameter.

The detection device 56 may be provided as a detection wire, or as guidewire or catheter.

As an option, the console 18 and the display 20 are provided. They are connected to the device 10 for renal artery measurement, indicated by lines 60, which may be wire connections or wireless connections. Further, at a least a data communication link 62 is provided between the device 10 for renal artery measurement and the detection device 56. For example, data from the sensor 58 is provided to the input unit 12.

In an option, it is further provided a medical imaging system 64 configured to acquire medical image data, as indicated with viewing angle 66, during the measuring of the at least one renal artery related parameter by the detection device 56. A line 68 indicates a data link, wireless or wire bound, between the device 10 for renal artery measurement and the medical imaging system 64. In another option, at least one marker 70 for the at least one sensor is provided, which marker is detectable by the medical imaging system 64. According to an option, the processing unit 14 is configured to determine a location of the at least one sensor 70 in relation to the renal artery based on the medical image data. The processing unit 14 is further configured to compare the determined location to at least one predefined location threshold for the reliability factor.

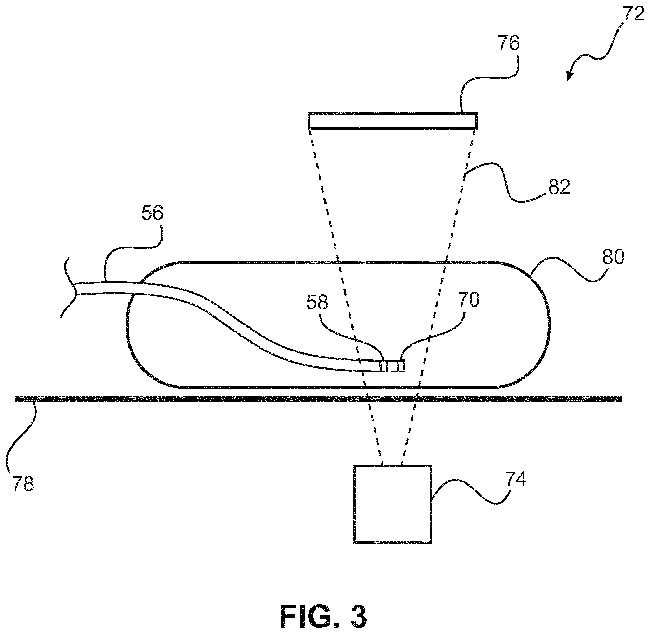

Fig. 3 shows an extract of an example of the system for renal artery measurement 50 comprising an X-ray imaging system 72 as the medical imaging system 64. The at least one marker 70 is radiopaque. For example, the X-ray imaging system 72 comprises an X-ray source 74 and an X-ray detector 76. The X-ray source 74 and the X-ray detector 76 may be movably supported on a C-arm structure, or may be movably supported by individual supports. A subject support table 78 may be provided to support a subject 80. The X-ray source 74 may be arranged below or above the subject support table 78 and the detector above or below the subject support table 78. An X-ray beam is indicated with dotted lines 82.

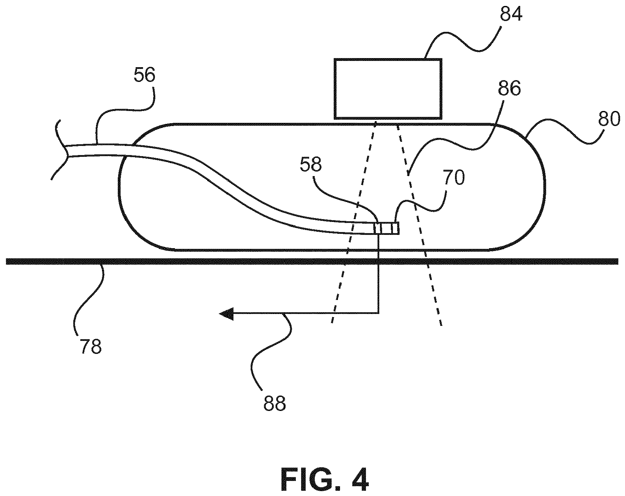

Fig. 4 shows an extract of an example of the system for renal artery measurement 50 comprising an ultrasound imaging system 84 as the medical imaging system 64. The at least one marker 70 is visible in ultrasound. An ultrasound beam is indicated with dotted lines 86.

It is noted that the medical imaging system being an X-ray imaging system or an ultrasound imaging system is provided as two options only.

In another option, a further imaging modality is provided for the medical imaging system.

In case of the X-ray imaging system or the ultrasound imaging system, the at least one marker is provided as a passive marker.

In an example, contrast agent may be provided, at least to a small extent, to render the vessel structure, i.e. the artery structure visible in X-ray imaging, like fluoroscopy. This facilitates segmenting the artery.

In another example, pre-acquired image data like 3D image data based on CT images is provided for the segmentation of the renal artery. In another option, anatomical landmarks are used to overlay pre-acquired image data with the current images.

In an example, provided as an option, the detection device comprises an ultrasound transducer that is provided as an active marker which detects ultrasound waves from an external ultrasound probe or transducer, e.g. the ultrasound imaging system 84, and which provides respective data, indicated with a signal arrow 88, to the processing unit 14. By combining the timing of the firing of ultrasound beams from the external probe and the signal being picked up by the transducer on the detection device, the location of the transducer in the ultrasound image can be displayed. In an alternative of this particular embodiment, although the sensor 58 and the marker 70 are illustrated separately in Fig. 4, the sensor and the marker can be one and the same component, for example an ultrasound transducer that measures flow velocity of blood in a vessel by using the Doppler effect, while also simultaneously having the role of an active marker which sends or receives ultrasound signal to or from an external ultrasound probe, e.g. the ultrasound imaging system 84, for location detection.

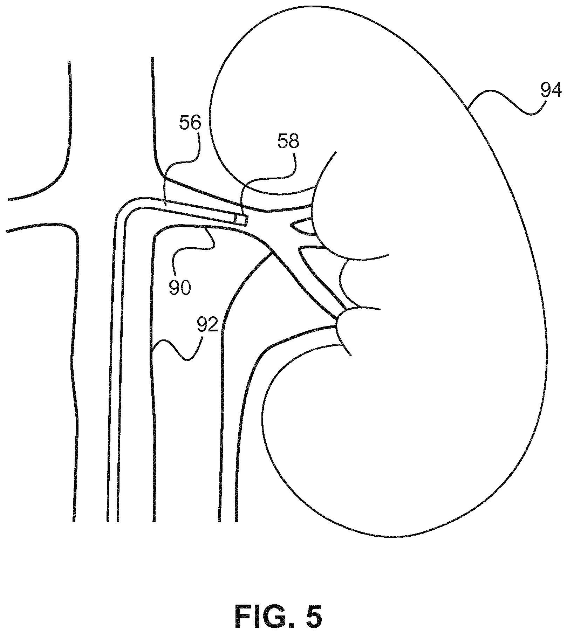

Fig. 5 shows an example of the detection device 56 inserted into a renal artery 90 of a subject via an aorta 92, from which the renal artery 90 branches off towards a kidney 94.

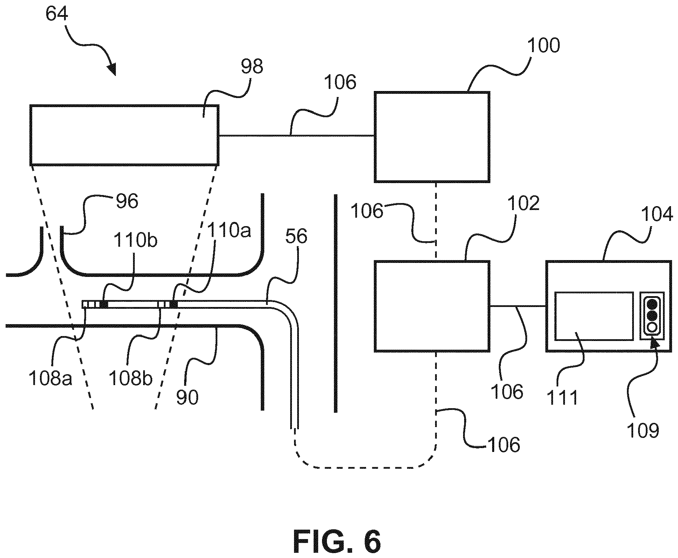

Fig. 6 shows a further example of the system 50 for renal artery measurement in an extract. The detection device 56 is shown in the context of the renal artery 90. A side-branch 96 is also indicated.

The system may comprise an X-ray detector 98 of an X-ray imaging system. Further, a control device 100 for operating the X-ray system may be provided. Still further, a device console 102 is provided and a display 104. The device console 102 is connected detection device 56. Data connections are indicated with connecting lines 106.

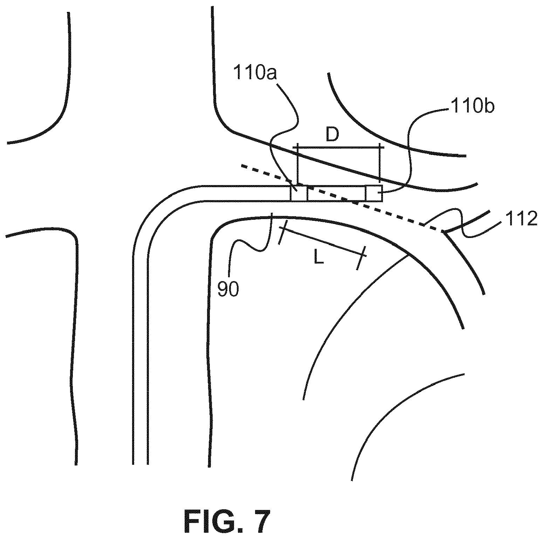

Fig. 6 and Fig. 7 show an example, in which the detection device 56 comprises two pressure sensors 108a, 108b spaced apart with a predetermined distance D, as also shown in Fig. 7. A marker 110a, 110b is provided for each of the two pressure sensors 108a, 108b, which markers 110a, 110b are detectable by the medical imaging system. The processing unit is configured to determine a projected distance L of the two pressure sensors in relation to a central axis 112 of the renal artery 90 based on the medical image data. The processing unit is configured to compare the projected distance *L* with the predetermined distance *D* for the reliability factor.

The detection device 56 is inserted into the renal artery to perform e.g. a dual-pressure PWV measurement.

The vessel walls of the corresponding renal artery section may be segmented, including the side branches, glomerulus, and closest portion of the aorta. The distance between the pressure sensors as projected on the axis of the renal artery is calculated.

The orientation of the device tip comprising the two sensors is thus determined and the orientation is brought into angular relation with the central axis of the renal artery to determine whether the device tip is correctly aligned.

In an example, a control device, or console, is connected to the intravascular device, to control the sensors and to receive the information from the sensors. The console provides time-stamps of the measurement outcomes and receives time-stamped X-ray images from a C-arm. For a measurement time point or period, the pressure signals are compared to generate and display a PWV value together with a recommendation on eligibility of this patient for renal denervation therapy, e.g. according to the European Patent Application EP18202488, filed on 25 October 2018, and incorporated by reference. In addition, the corresponding X-ray image is analyzed by identifying the radiopaque markers. With the aid of this data, the device is segmented from the X-ray image and the location of the pressure sensors is derived.

By comparing the detected relative distance in vessel direction with the known distance of the two sensors, it is provided that a measurement from such a situation, where the two sensors are positioned at an angle to the axis of the vessel, and the two sensors may therefore not properly detect the time for the pressure/flow pulse to move from the first sensor to the second sensor as the distance travelled by the pulse is not the distance between the sensors, is indicated. For example, when the distance between the sensors is 2.00 cm, the diameter of the renal artery is 5 mm, and the tip of the device is at an angle to the axis of the vessel and the vessel walls, the actual distance travelled could be down to 1.93 cm, a systemic error of more than 3%. This deviation is increased in case the device is bent between the two sensors, since the device will by design be flexible in order to navigate through the vascular structure. For example, if a bend in the device between the two sensors equals a 120° angle, this causes an error of 17% in the assumed distance travelled by the pulse wave. It is thus ensured, by providing the reliability factor, that actually incorrect measurements are not forming a basis for further steps.

An indicator 109 may be provided for indicating the reliability factor. For example, a red-yellow-green light coding may be applied to display if the reliability factor is indicating a value that is not acceptable, acceptable to a certain extent, or completely acceptable. For example, the background to the recommendation is highlighted with a respective color. As an option, in addition or alternatively, a field 111 may be provided with text, number or symbolic information. For example, an indication of the PWV value may be provided with some indication of the findings.

For example, if this distance deviates less than e.g. 5% from the theoretical value of 2 cm, a signal is displayed on the display of the console that the generated value and recommendation are of high quality, e.g. a green light. If the distance deviates e.g. 5% or more from the theoretical value of 2 cm, a further signal is displayed on the console display that the generated value and recommendation are of medium quality, e.g. a yellow light. If the deviation is e.g. 10% or more, an indicator of low quality is displayed, such as a red light.

For example, a device is positioned in the renal artery. A console controls the pressure sensors and receives pressure data. Based on this information, renal artery stiffness and PWV are calculated. Simultaneously, the renal artery is imaged by X-ray. The images are transmitted to the device console. Here, the position of the sensors with respect to the renal artery is determined for the time in which the PWV is calculated. This provides a quality metric that is displayed to the user alongside the PWV calculation and the therapy recommendation.

Hence, the current image is used for determining if the detection device is arranged in an angle to the vessel.

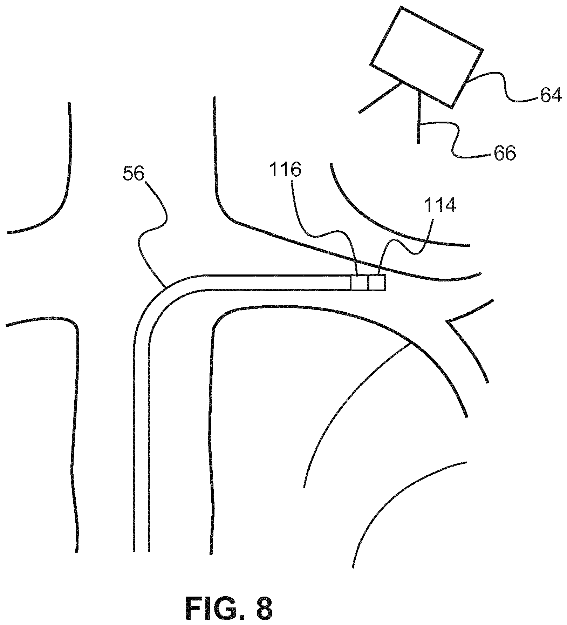

Fig. 8 shows an example in which the detection device 56 comprises a flow sensor, e.g. a flow sensor or a flow velocity sensor, which sensor is indicated with reference numeral 114. Further, a marker 116 is provided for the flow sensor 114, which marker 116 is detectable by the medical imaging system 64. The processing unit is configured to determine, based on the medical image data acquired during the measuring of the at least one renal artery related parameter, a relative distance of the flow sensor in relation to a central axis of the renal artery and/or in relation to a wall of the renal artery based on the medical image data. The processing unit is also configured to compare the relative distance with a predetermined distance value for the reliability factor.

In an option, at least two markers are provided near the tip to deduce the orientation of the tip compared to the vessel axis.

The image processing determines whether the flow sensor is located close to vessel wall and oriented away from the center of the vessel. If so, the reliability of the measurement is low.

In an example, X-ray imaging or CT imaging or ultrasound imaging may be provided to detect the position of the flow sensor. For segmenting the vessel, contrast agent, pre-acquired CT data or anatomical landmarks in combination with a pre-acquired vessel may can be applied.

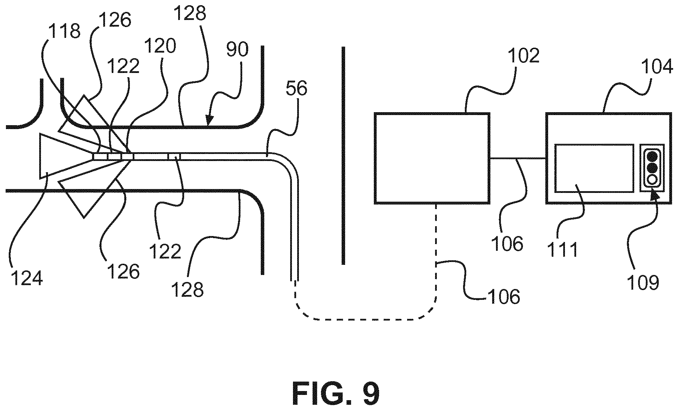

Fig. 9 shows an example in which the detection device 56 comprises a flow sensor 118 and at least one sensor 120 that measures distance values. The processing unit is configured to determine a distance of the flow sensor 118 to the renal artery wall during the flow measurement. The processing unit is also configured to compare the distance with a predetermined distance value.

In an option, one pressure sensor 122 is provided in addition. In another option, two pressure sensors 122 are provided.

As a further option, the least one sensor 120 that measures distance values is provided as an intravascular ultrasound sensor (IVUS).

As an example, if a device with at least one Doppler flow sensor is positioned so that the Doppler flow sensor is aimed away from the center of the vessel, the flow velocity measurement does not detect the maximum flow velocity, but a lower value. Blood flow in a vessel has a velocity profile over the diameter of the vessel with lower velocities near the vessel walls. A measurement near or too close to the wall would lead to unreliable and/or inaccurate flow measurement outcomes in renal arteries, in particular since the renal artery is quite large in diameter compared to typical intravascular device dimensions and thus have more space for misalignment of the Doppler flow sensor.

In Fig. 9, the detection device 56 is positioned in the renal artery 90. The console 102 controls the flow sensor 118 and the pressure sensors 122 and receives flow and pressure data. Based on this information, renal artery stiffness and pulse wave velocities (PWV), renal blood flow, and/or resistance, are calculated. A first field or viewing direction 124 for measuring is indicating the measuring direction of the flow sensor 118. Simultaneously, the renal artery is imaged by the IVUS. Two second fields or viewing directions 126 for measuring are indicating the measuring direction of the IVUS. The images are transmitted to the device console 102. Here, the position of the sensors with respect to a renal artery wall 128 is determined for the time in which the PWV is calculated. This provides a quality metric that is displayed to the user alongside the PWV calculation and the therapy recommendation on the display 104. In Fig. 9, the flow sensor is shown aligned within the center of the renal artery 90, and the reliability of the measurement outcome is thus high.

Hence, external imaging is not required. For example, a flow sensor is provided on the tip of the detection device looking in the forward direction for flow sensing, and two distance measuring sensors are attached in the vicinity looking sideward to the vessel walls. A further sensor may be provided as a pressure sensor on the detection device in a more proximal direction.

In an example, one or more of the sensors is a flow sensor, and IVUS sensor(s) is/are incorporated on the device next to the flow sensor(s). In another example, an IVUS catheter is advanced over the flow-sensing device, e.g. the flow-sensing guide wire. In other words, the IVUS is provided integrated on the flow-measuring device and also in a separate form.

In an example, the IVUS is angled towards the same measurement volume as interrogated by the flow sensor.

In another example, the IVUS has a viewing direction that is perpendicular to the device.

From the IVUS sensor images, e.g. a phased array IVUS, the proximity of the IVUS sensor (and thus the flow sensor) to the vessel wall is determined, and the estimated distance of the volume that is used for Doppler flow measurement to the vessel wall. If this is below a threshold, e.g. 0.5 mm, or 1 mm, the measurement outcome reliability is classified as "medium". If the distance is below a second threshold (e.g. 1 mm or 2 mm) the measurement outcome reliability is classified as "low".

For example, a device is positioned in the renal artery. The console controls the flow and pressure sensor and receives flow and pressure data. Based on this information, renal artery stiffness and PWV are calculated. Simultaneously, the renal artery is imaged by IVUS towards the side walls, whereas the flow sensor analyses in a forward direction. The images are transmitted to the device console. Here, the position of the sensors with respect to the renal artery wall is determined for the time in which the PWV is calculated. This provides a quality metric that is displayed to the user alongside the PWV calculation and the therapy recommendation. In this case, the flow sensor is aligned nicely within the center of the renal artery, and the reliability of the measurement outcome is high

In another example, not shown in detail, a medical imaging system is provided that acquires medical image data during the measuring of the at least one renal artery related parameter. The medical image data comprises structural anatomical information of the renal artery comprising at least one of the group of: branching off location from the aorta, side branches and connecting location with the glomerulus. The processing unit is configured to determine the location of the at least one sensor in relation to the renal artery based on the medical image data. The processing unit is further configured to compare the determined location to at least one predefined location for the reliability factor.

Hence, in addition, the X-ray, ultrasound and/or IVUS images are analyzed for position of the sensors along the renal artery, taking into account a distance to side branches, the glomerulus, and the aorta. If one of the sensors is located closely to a side branch, the glomerulus, the ostium of the renal artery, or the aorta, the measurement outcome reliability is classified as "low".

As another example, if a device is positioned not deeply enough within the renal artery, i.e. navigated not far enough towards the kidney, a sensor that is incorporated at the tip may provide measurement from the aorta environment or the ostium of the renal artery, and not the renal artery itself. This would lead to outcomes that may be interpreted erroneously. For example, in a device with two sensors, one sensor may measure in the renal artery ostium that has a significantly larger diameter than the vessel at the location of the second sensor. This gives false outcomes in a measurement that correlates e.g. pressure to vessel diameter to determine the arterial stiffness or PWV.

Further, if the device is positioned too deep within the renal artery, e.g. close to a bifurcation or past a bifurcation within the renal artery, pressure/pulse wave reflections from bifurcations may be more difficult to filter out from the primary signal and may lead to erroneous results.

The medical imaging system may be provided as the above-mentioned X-ray imaging system or the also mentioned ultrasound imaging system or an imaging sensor on the detection device. In another example, another imaging modality, like magnetic resonance (MRT-) imaging is provided. In a further example, the medical imaging system is provided as an IVUS on the device itself.

The medical imaging system is provided in order to provide imaging and thus orientation along the vessel direction.

In another example, not shown in detail, the measurement arrangement is configured to measure over a period of time that incorporates a predetermined number of heartbeats, and to provide measurements for sub-periods of the period of time. The processing unit is configured to determine a variation between measurements of different sub-periods. The processing unit is also configured and to compare the variation to at least one predefined variation threshold.

As an option, a medical imaging system is not required, but the measurement can be based on the intravascular device itself.

In an example, the measurement arrangement is configured to measure values for sub-periods of the period of time. The processing unit is configured to determine a variation between values of different sub-periods; and to compare the variation with at least one predefined variation threshold.

Hence, the reliability is determined from the information from the sensors on the device themselves. There are no additional markers or sensors required on the intravascular device, and there is no need for external imaging. The measurement is performed over a period of time that incorporates a set number of heartbeats (x). The number of heartbeats in the calculation set can be easily inferred from the sensor signals. In case the heartbeat or heart rate is difficult to discern, this is already an indication for the quality grade of the measurement.

For example, the pressure value(s), flow value(s), and/or calculated arterial stiffness or PWV value are calculated per heartbeat or set number of heartbeats (y), in which x >> y. Then, the variation between the values from different sub-periods in the measurement period is determined. If the variation is above a threshold, e.g. CV (coefficient of variation, i.e. standard deviation compared to mean value) = 2%, the measurement outcome reliability is classified as "medium". If the distance is above a second threshold, e.g. CV is 5%, the measurement outcome reliability is classified as "low". A variation in the values indicates that for instance a flow sensor is located too closely to the vessel wall and provides varying results, or a sensor is located too closely to the glomerulus causing artefacts in the signal processing from reflections

Briefly said, the signal, i.e. the value, is determined over the heartbeat cycle, and if the outcome is the same over time, this is an indication for a reliable measurement.

In another example, the measurement arrangement is configured to measure curves for sub-periods of the period of time. The processing unit is configured to determine a variation between curves of different sub-periods; and to compare the variation to at least one predefined variation threshold.

Briefly said, the wave form, also referred to as the spectrum of the signal, is determined over the heartbeat cycle, and if the outcome in form of the wave form is the same over time, this is an indication for a reliable measurement.

In an example, a curve from one sensor is compared to a curve from the same sensor over time.

Regarding a comparison of flow signals from, for example, four consecutive heartbeats, the flow spectra are nearly identical, if the sensor is likely placed well. Instead of four heartbeats, also a lower or higher number of heartbeats can be provided. The measurement outcome is likely correct. If the flow spectra are detectably different, the sensor is likely not placed well, and the measurement outcome is thus likely not correct.

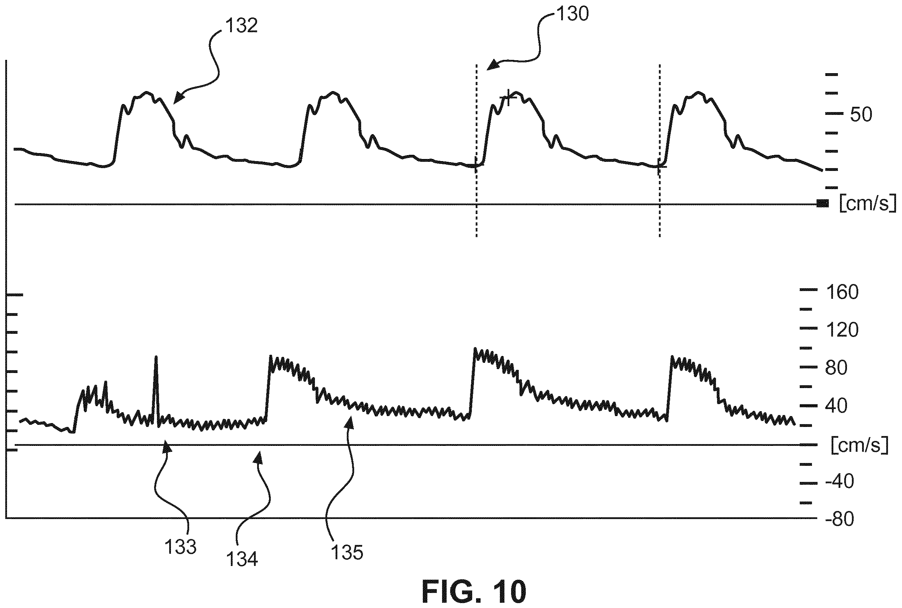

In another example, in addition to the flow curve or flow spectra, consistency in the IPV (instantaneous peak velocity) tracing (the curve of the spectra tracing in the upper part of Fig. 10) is checked. For this signal, similar conditions apply as for pressure signals, i.e. an assessment is made concerning a correlation across consecutive heartbeats, e.g. for the part of the signal that is used for PWV assessment, which is e.g. the upward flank in the signal/curve.

In an example, the curves are referred to as spectra of the measurements.

Fig. 10 shows an example of a comparison 130 of flow signals from four consecutive heartbeats. In the upper part of Fig. 10, a first flow spectrum is indicated with a first curve 132. As can be seen, the flow spectra for the consecutive heartbeats are nearly identical. The sensor is thus likely placed well, and the measurement outcome is likely correct. In the lower part of Fig. 10, a second first flow spectrum is indicated with a second curve 134. As can be seen, the flow spectra for the consecutive heartbeats are detectably different, as indicated with first arrow 133 and second arrow 135. The sensor is thus likely not placed well, and the measurement outcome is thus likely not correct. The first and the second curves are shown across a horizontal axis indicating the time, and a vertical axis indicating an intensity, for example with units cm/s.

In another example, the measurement arrangement is configured to perform two measurements. The processing unit is configured to determine a variation of the values for the two measurements. The processing unit is also configured to compare the variation to at least one predefined variation threshold.

This example does not require a medical imaging system, but can be based on the intravascular device itself.

In an example, for the variation of the values for the two measurements, the processing unit is configured to determine a variation between measurements of different sub-periods; and to compare the variation to at least one predefined variation threshold.

In an example, at least two identical sensors are provided, and the measurement arrangement is configured to perform the measuring for each sensor over a period of time that incorporates at least one cardiac cycle. The processing unit is configured to determine a variation in the reproducibility between the measurements of the two sensors; and to compare the variation to at least one predefined variation threshold. Two curves are hence compared.

The two sensors are configured to measure the same vessel physiology parameter, e.g. two pressure sensors, or two flow sensors, or two IVUS sensors. If correctly positioned, the spectra, i.e. the curves should overlap.

For each heartbeat or number of heartbeats, the spectra of the signals over the heartbeat is compared and analyzed for reproducibility over the measurement period. A significant deviation is an indicator of one of the sensors being in the aorta or ostium as the local wall stiffness will be different, close to a bifurcation, or past a bifurcation as the pulse wave reflections will be different or absent; or that a flow sensor is not interrogating the center portion of the blood vessel.

Regarding a comparison of pressure wave pulse signals from two pressure sensors, if the pressure pulse is nearly identical, the sensors are thus likely placed well, and the measurement outcome is likely correct. If the pressure pulses are detectably different, the sensors are likely not placed well, and the measurement outcome is thus likely not correct.

In a further example, similar applies to IPV tracing in case flow sensors are used. If signals are nearly identical, the sensors are thus likely placed well. If the signals are detectably different, the sensors are likely not placed well, and the measurement outcome is thus likely not correct.

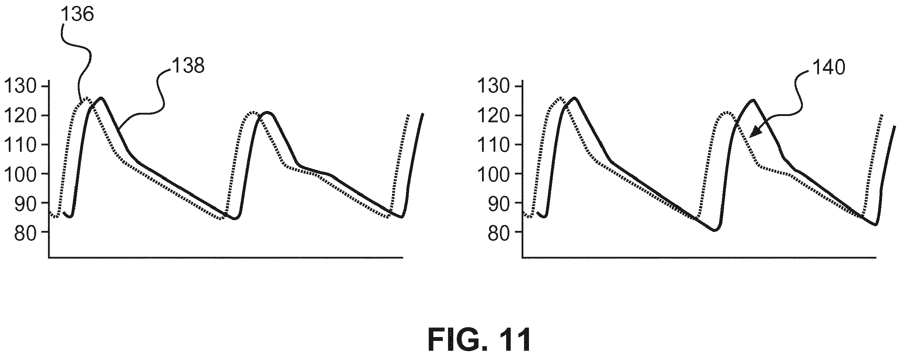

Fig. 11 shows a comparison of pressure wave pulse signals from two pressure sensors. A first pressure curve 136 and a second pressure curve 138 are indicated. On the left part of Fig. 11, the pressure pulse is nearly identical in amplitude and shape. The sensors are thus likely placed well, and the measurement outcome is likely correct. On the right part of Fig. 11, the pressure pulses are detectably different, as indicated by pointer arrow 140. The sensors are thus likely not placed well, and the measurement outcome is thus likely not correct. The first and the second curves are shown across a horizontal axis indicating the time, and a vertical axis indicating an arterial pressure intensity, for example with units mmHg.

The two curves are provided in an offset and can be shifted for correction of the time differences, as the two sensors are not at the same location.

In another example, the measurement arrangement is configured to perform the measuring over a period of time that incorporates a predetermined number of heartbeats. Measurements are provided for sub-periods of the period of time. The processing unit is configured to determine a variation between the measurements and stored reference curves. The processing unit is also configured to compare the variation to at least one predefined variation threshold.

The measurement does not require a medical imaging system but can be based on the intravascular device itself.

Hence, two curves are compared. For example, the measured curve is compared with a curve from an ideal position.

Briefly said, the shape of the spectra of the pressure and flow signals over the heartbeats are compared to a library of "good" spectra. A deviation of the spectra indicates an unreliable measurement due to an unfavorable location of the sensor during the measurement.

In an example, not further shown in detail, three sensors are provided comprising at least one of the group of pressure sensor and flow sensor. The processing unit is configured to calculate a renal artery related parameter based on different combinations of the three sensors. The processing unit is also configured to determine a variation of the calculated pulse wave velocities. The processing unit is further configured to compare the variation to at least one predefined variation threshold.

This allows for multiple calculations of arterial stiffness or PWV from varying combinations of sensors, which can be averaged to derive the value to be displayed. A discrepancy between the outcomes for a single measurement period above a set threshold indicates a low reliability of the outcome.

By providing three sensors, three different ways for determining pulse wave velocity are provided, since the signals, i.e. measures, from sensors one and two, or from sensors two and three or from sensors one and three can be used to determine the respective pulse wave velocity. The pulse wave velocities are calculated for comparing the outcome of the calculations, and thus the result of the measurements. Hence, additional comparison steps are provided. One example for the renal artery related parameter is pulse wave velocity (PWV).

In another example, also not further shown in detail, at least two sensors are provided and the measurement arrangement is configured to perform the measuring for a first position and a second position being different from the first location. For example, the pull back of the intravascular device is provided over a series of measurement periods. The processing unit is configured to calculate a pulse wave velocity (PWV) for the first and the second location. The processing unit is also configured to determine a variation of the calculated pulse wave velocities. The processing unit is furthermore configured to compare the variation to at least one predefined variation threshold.

Thus, multiple calculations are compared. The outcomes of the calculations and thus the measurements should be identical if the detection device is in the straight part of the renal artery.

For achieving the two different positions, a pullback device can be provided that moves the detection device in the artery from the first to the second location, wherein the first location is in viewing direction in front of the second position, i.e. the first position is closer to the kidney and the second position is closer to the aorta. In other words, the first positon is the distal positon of the two positions, and the second position is the proximal position of the two positions. Alternatively, the device is pushed forward and the location of the two positions is reversed.

If the sensors are positioned correctly, the value will remain constant during a few millimeters or centimeters pullback or push-forward, and then will change as one of the sensors is pulled out of the renal artery, or moves too closely to the vessel wall. On the other hand, if the device was positioned too deeply within the renal artery, the value will first quickly change before reaching a constant period. If the device was positioned not deep enough, the value will quickly change but not reach a constant period. If a flow sensor was located unfavorably with respect to the vessel wall and vessel center, the value will also vary strongly during the pullback. This can also be used to identify a portion of the pullback in which the outcome is reliable, even if the value was not constant at first. The pullback can be manual, e.g. with analysis of pullback speed by imaging or other means, or assisted by a device for a more controlled pullback speed.

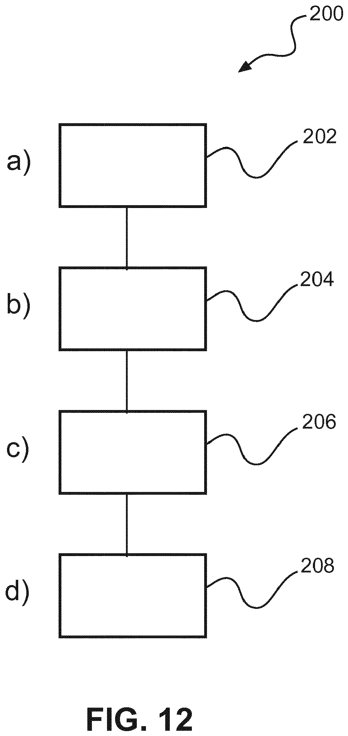

Fig. 12 shows steps of an example of a method 200 for renal artery measurement. The method 200 comprises the following steps:
- In a first step 202, also referred to as step a), at least one renal artery related parameter is measured with a detection device inserted in the renal artery.
- In a second step 204, also referred to as step b), location information of the detection device is provided, which location information is related to a position of the detection device inside the renal artery during the measuring.
- In a third step 206, also referred to as step c), a reliability factor for the measuring is determined based on the location information.
- In a fourth step 208, also referred to as step d), the reliability factor for the measured at least one renal artery related parameter is provided.

In an example, the at least one renal artery related parameter comprises at least a flow measurement of blood flow in the renal artery.

In an example, the at least one renal artery related parameter comprises a pressure measurement of blood pressure in the renal artery.

In a further example, the at least one renal artery related parameter comprises a vessel diameter measurement of the renal artery.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims. In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for assessment of reliability of vessel physiology related measurement, the device comprising:
- an input unit (12);
- a processing unit (14); and
- an output unit (16);
wherein the input unit is configured to receive at least one vessel physiology related measurement from a detection device (56) inserted into a vessel of a subject; and to receive location information of the detection device, which location information is related to a position of the detection device inside the vessel during the measurement;
wherein the processing unit is configured to determine a reliability factor for the at least one vessel physiology related measurement based on the location information; and
wherein the output unit is configured to provide the reliability factor for the at least one vessel physiology related measurement.

2. A system (50) for assessment of reliability of vessel physiology related measurement, comprising:
- a device (10) according to claim 1; and
- a measurement arrangement (54) comprising a detection device (56) for insertion into a vessel of a subject, the detection device having at least one sensor (58) configured to provide the at least one vessel physiology related measurement.

3. System according to claim 2, wherein it is further provided:
- a medical imaging system (64) configured to acquire medical image data during the at least one vessel physiology related measurement; and
- at least one marker (70) for the at least one sensor, which marker is detectable by the medical imaging system;
wherein the processing unit is configured to determine the location of the at least one sensor in relation to the vessel based on the medical image data; and to compare the determined location to at least one predefined location threshold for determining the reliability factor; and
i) wherein the medical imaging system is an X-ray imaging system (72) and the at least one marker is radiopaque; or
ii) wherein the medical imaging system is an ultrasound imaging system (84) and the at least one marker is visible in ultrasound.

4. System according to claim 3, wherein the detection device comprises an ultrasound transducer; and
wherein the transducer is provided as an active marker that detects ultrasound waves from an external ultrasound probe.

5. System according to claim 3 or 4, wherein the detection device comprises two pressure sensors (108a, 108b) spaced apart with a predetermined distance;
wherein a marker (110a, 110b) is provided for each of the two pressure sensors, which markers are detectable by the medical imaging system;
wherein the processing unit is configured to determine a projected distance of the two pressure sensors in relation to a central axis of the vessel based on the medical image data; and
wherein the processing unit is configured to compare the projected distance with the predetermined distance for determining the reliability factor.

6. System according to one of the claims 3 to 5, wherein the detection device comprises a flow sensor (114);
wherein a marker (116) is provided for the flow sensor, which marker is detectable by the medical imaging system; and
wherein the processing unit is configured to determine, based on the medical image data acquired, a relative distance of the flow sensor in relation to a central axis of the vessel and/or in relation to a wall of the vessel based on the medical image data; and to compare the relative distance with a predetermined distance value for determining the reliability factor.

7. System according to one of the claims 2 to 6, wherein the detection device comprises a flow sensor (118) and at least one sensor (120) that measures distance values; and
wherein the processing unit is configured to determine a distance of the flow sensor to the vessel wall during the flow measurement; and to compare the distance with a predetermined distance value.

8. System according to one of the claims 3 to 7, wherein the at least one vessel physiology related measurement is a renal artery physiology related measurement, wherein the medical imaging system is provided that acquires medical image data during the renal artery physiology related measurement, wherein the medical image data comprises structural anatomical information of the renal artery comprising at least one of the group of: branching off location from the aorta, side branches and connecting location with the glomerulus; and
wherein the processing unit is configured to determine the location of the at least one sensor in relation to the renal artery based on the medical image data; and to compare the determined location to at least one predefined location for the reliability factor.

9. System according to one of the claims 2 to 8, wherein the measurement arrangement is configured to measure over a period of time that incorporates a predetermined number of heartbeats; and to provide measurements for sub-periods of the period of time; and
wherein the processing unit is configured to determine a variation between measurements of different sub-periods; and to compare the variation to at least one predefined variation threshold.

10. System according to one of the claims 2 to 9, wherein the measurement arrangement is configured to perform two measurements; and
wherein the processing unit is configured to determine a variation of the values for the two measurements; and to compare the variation to at least one predefined variation threshold.

11. System according to one of the claims 2 to 10, wherein the measurement arrangement is configured to perform the measurement over a period of time that incorporates a predetermined number of heartbeats; wherein measurements are provided for sub-periods of the period of time; and
wherein the processing unit is configured to determine a variation between the measurements and stored reference curves; and to compare the variation to at least one predefined variation threshold.

12. System according to one of the claims 2 to 11, wherein three sensors are provided comprising at least one of the group of pressure sensor and flow sensor; and
wherein the processing unit is configured to calculate pulse wave velocities from vessel physiology related measurements based on combinations of different measurements provided by the three sensors; to determine a variation of the calculated pulse wave velocities; and to compare the variation to at least one predefined variation threshold.

13. System according to one of the claims 2 to 11, wherein at least two sensors are provided and the measurement arrangement is configured to perform the measurement for a first location and a second location being different from the first location; and the processing unit is configured to calculate a pulse wave velocity (PWV) for the first and the second location; to determine a variation of the calculated pulse wave velocities; and to compare the variation to at least one predefined variation threshold.

14. A method (200) of assessing reliability of vessel physiology related measurement, comprising the following steps:
a) providing (202) at least one vessel physiology related measurement with a detection device inserted into a vessel;
b) providing (204) location information of the detection device, which location information is related to a position of the detection device inside the vessel during the measurement;
c) determining (206) a reliability factor for the vessel physiology related measurement based on the location information; and
d) providing (208) the reliability factor for vessel physiology related measurement.

15. A computer program element for controlling a device according to claim 1, which, when being executed by a processing unit, is adapted to perform the method steps of claim 14.
